# EUROPEAN PATENT APPLICATION

(11) **EP 2 821 015 A1**
(43) Date of publication of application: **07.01.2015**
(21) Application number: 14159938.1
(22) Date of filing: 14.03.2014
(51) Int. Cl.: A61B 8/08, A61B 8/00, G06F 19/00

(54) **Ultrasound system and method of providing reference image corresponding to ultrasound image**

(30) Priority: 24.06.2013 KR 20130072488
(71) Applicant: Samsung Medison Co., Ltd., Gangwon-do 250-870 (KR)
(72) Inventor: Lee, Jun-kyo, Gangwon-do (KR)
(74) Representative: Schmid, Wolfgang

(57) **Abstract**

Provided are an ultrasound system and a method of providing a reference image corresponding to an ultrasound image of a target. The ultrasound system includes: a storage unit configured to store a plurality of images corresponding to respective targets; and a processor configured to generate an ultrasound image which depicts the target included in a subject by using ultrasound data acquired from the subject, extract at least one reference image corresponding to the ultrasound image from among the plurality of images, and control a display of the reference image extracted.

## Description

### RELATED APPLICATIONS

This application claims the benefit of Korean Patent Application No. 10-2013-0072488, filed on June 24, 2013, in the Korean Intellectual Property Office, the disclosure of which is incorporated herein in its entirety by reference.

### BACKGROUND

### 1. Field

One or more embodiments of the present invention relate to ultrasound systems, and more particularly, to ultrasound systems and methods of providing a reference image corresponding to an ultrasound image.

### 2. Description of the Related Art

Since ultrasound systems have noninvasive and nondestructive characteristics, they are widely used in medical treatment for obtaining information from a subject. Such systems are very important because they may provide medical practitioners with real-time high-resolution images of internal tissues of a subject without performing a surgical operation by directly incising and observing inner parts of the subject.

An ultrasound system transmits an ultrasound signal to a subject including a target (for example, a liver, an unborn child, or a bloodstream) and receives an ultrasound signal (i.e., an ultrasound echo signal) reflected from the subject to thereby generate an ultrasound image.

Whether the target is normal is determined based on the ultrasound image, and the accuracy of determination may depend on the degree of skill of a medical practitioner. Also, even when provided with the ultrasound image, a patient may have difficulties in understanding whether the target is normal.

### SUMMARY

One or more embodiments of the present invention include ultrasound systems and methods of storing a plurality of normal reference images and a plurality of abnormal reference images corresponding to a plurality of applications in a storage unit, extracting at least one reference image from the storage unit, from among a normal reference image and an abnormal reference image corresponding to an ultrasound image of a target including the target, and providing the at least one reference image.

Additional aspects will be set forth in part in the description which follows and, in part, will be apparent from the description, or may be learned by practice of the presented embodiments.

According to one or more embodiments of the present invention, an ultrasound system includes: a storage unit configured to store a plurality of images corresponding to respective targets; and a processor configured to generate an ultrasound image which depicts the target included in a subject by using ultrasound data acquired from the subject, extract at least one reference image corresponding to the ultrasound image from among the plurality of images, and control a display of the reference image extracted.

According to one or more embodiments of the present invention, a method of providing a reference image includes: generating an ultrasound image which depicts the target included in a subject by using ultrasound data acquired from the subject; extracting at least one reference image corresponding to the ultrasound image from among a plurality of images stored in a storage unit configured to store the plurality of images corresponding to respective targets; and controlling a display of the reference image extracted.

### BRIEF DESCRIPTION OF THE DRAWINGS

These and/or other aspects will become apparent and more readily appreciated from the following description of the embodiments, taken in conjunction with the accompanying drawings in which:
FIG. 1 is a block diagram illustrating a configuration of an ultrasound system according to an embodiment of the present invention;
FIG. 2 is a block diagram illustrating a configuration of an ultrasound data acquiring unit according to an embodiment of the present invention;
FIG. 3 is a flowchart illustrating a process of providing a reference image and measurement result information, according to an embodiment of the present invention;

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

Hereinafter, embodiments of the present invention will be described in detail with reference to the accompanying drawings.

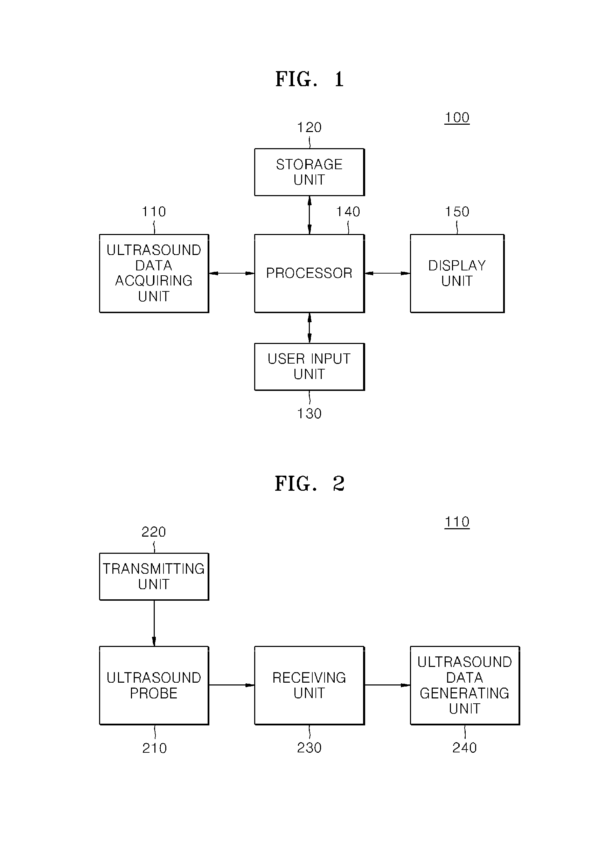

FIG. 1 is a block diagram illustrating a configuration of an ultrasound system according to an embodiment of the present invention. Referring to FIG. 1, an ultrasound system 100 includes an ultrasound data acquiring unit 110, a storage unit 120, a user input unit 130, a processor 140, and a display unit 150.

The ultrasound data acquiring unit 110 transmits an ultrasound signal to a target. The target may include, for example, various organs such as a heart, an unborn child, and a bloodstream. Also, the ultrasound data acquiring unit 110 receives an ultrasound signal (i.e., an ultrasound echo signal) reflected from the target to thereby acquire ultrasound data corresponding to an ultrasound image.

FIG. 2 is a block diagram illustrating a configuration of the ultrasound data acquiring unit 110 according to an embodiment of the present invention. Referring to FIG. 2, the ultrasound data acquiring unit 110 includes an ultrasound probe 210, a transmitting unit 220, a receiving unit 230, and an ultrasound data generating unit 240.

The ultrasound probe 210 includes a plurality of transducer elements (not illustrated) that convert an electrical/ultrasound signal into an ultrasound/electrical signal. The ultrasound probe 210 transmits an ultrasound signal to the target and receives an ultrasound echo signal reflected from target to thereby generate an electrical signal (hereinafter referred to as a reception signal). The reception signal is an analog signal. For example, the ultrasound probe 210 includes a convex probe, a linear probe, and a three-dimensional (3D) probe.

The ultrasound data acquiring unit 110 includes the transmitting unit 220. The transmitting unit 220 controls transmission of the ultrasound signal. Also, the transmitting unit 220 includes a plurality of pulsers (not illustrated) that generate a plurality of pulse signals. In consideration of a transducer element and a focus point, the transmitting unit 220 delays by a predetermined period the pulse signals output from the pulsers to thereby output a transmission pulse signal with a predetermined transmission profile. The ultrasound probe 210 generates and outputs an ultrasound signal in response to the transmission pulse signal received from the transmitting unit 220. The ultrasound probe 210 may output the ultrasound signal by forming a beam in a predetermined direction (for example, a scan line) according to the profile of a transmission pulse. Also, the ultrasound probe 210 receives the ultrasound echo signal reflected from the target to thereby output an electrical reception signal.

The receiving unit 230 converts the reception signal received from the ultrasound probe 210 into a digital signal. Also, in consideration of the transducer element and the focus point, the receiving unit 230 performs reception beamforming on the digital signal to generate a reception focus signal. In an embodiment, the receiving unit 230 may perform the reception beamforming by using an application specific integrated circuit (ASIC) in which physical delay units such as resistors are integrated, or by using a digital signal processor (DSP) operating based on a software algorithm. Since the reception beamforming may be performed by various well-known methods, a detailed description thereof will be omitted herein.

The ultrasound data generating unit 240 generates, by using the reception focus signal received from the receiving unit 230, ultrasound data corresponding to the ultrasound image. The ultrasound data includes radio frequency (RF) data. However, the ultrasound data is not limited thereto. Also, the ultrasound data acquiring unit 110 may perform various signal processing (for example, gain control) on the reception focus signal to thereby generate the ultrasound data. In an embodiment, the ultrasound data acquiring unit 110 may be implemented by one or more DSPs.

In the above embodiment, it has been described that the ultrasound data acquiring unit 110 transmits the ultrasound signal to the target, receives the ultrasound echo signal reflected from the target, and acquires the ultrasound data corresponding to the ultrasound image. However, in another embodiment, the ultrasound data acquiring unit 110 may receive the ultrasound data from an external device (not illustrated) that is connected to the ultrasound system 100 in a wired or wireless manner.

Referring to FIG. 1, the storage unit 120 stores the ultrasound data acquired by the ultrasound data acquiring unit 110. Also, the storage unit 120 stores a plurality of images corresponding to each of a plurality of targets. The target corresponds to an organ to be diagnosed (i.e., a target of which ultrasound image is to be formed) from among a plurality of organs included in a subject (i.e, a patient). For example, if a user wants to diagnose a liver included in the subject, the target corresponds to the liver.

In an embodiment, with respect to each of the targets, the storage unit 120 stores a plurality of first images acquired when each target is normal, and a plurality of second images acquired when each target is abnormal. Also, with respect to each of the first images, the storage unit 120 may store diagnosis information about a diagnosis result, and medical information such as a medical treatment corresponding to the diagnosis result, and a plurality of progress images representing a change in the target according to the progress of the medical treatment. According to an embodiment, the first images, the diagnosis information, the medical information, and the progress images may be stored in the form of a mapping table. For example, the storage unit 120 includes a random access memory (RAM), a nonvolatile RAM (NVRAM), a flash memory, a compact disk, and a magnetic or optical data storage device.

In the above embodiment, it has been described that a plurality of images (i.e., the first images and the second images) are stored in the storage unit 120 of the ultrasound system 100. However, in another embodiment, a plurality of images (i.e., the first images and the second images) may be stored in an external storage device (not illustrated) that is connected to the ultrasound system 100 in a wired or wireless manner.

The user input unit 130 receives from a user an input of various user commands for driving the ultrasound system 100. In an embodiment, the user commands include a selection command for selecting at least one of the images. As an example, the user commands include a selection command for selecting at least one of the first images. As another example, the user commands include a selection command for selecting at least one of the second images. As another example, the user commands include a selection command for selecting at least one of the first images and at least one of the second images. Also, the user commands include a target selection command for selecting at least one of the target. In an embodiment, the user commands include a command for providing, when a first referene image of the first images is selected, at least one of the diagnosis information corresponding to the selected first reference image, the corresponding medical information, and the corresponding progress images. However, the user commands are not limited thereto. For example, the user input unit 130 includes a control panel, a track ball, a keyboard, a touchscreen, and a mouse.

The processor 130 is connected to the ultrasound data acquiring unit 110, the storage unit 120, and the user input unit 130. For example, the processor 140 includes a central processing unit (CPU), a microprocessor, and a graphic processing unit (GPU).

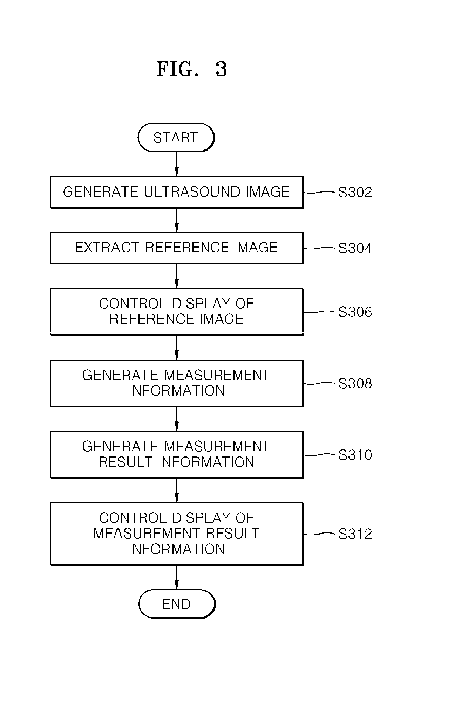

FIG. 3 is a flowchart illustrating a process of providing a reference image together with an ultrasound image, according to an embodiment of the present invention. Referring to FIG. 3, the processor 140 generates an ultrasound image by using the ultrasound data received from the ultrasound data acquiring unit 110 (S302). For example, the ultrasound image includes a brightness mode (B mode) image, a spectral Doppler mode (D mode) image, a color Doppler mode (C mode) image, and an elasticity image.

By using a probability model, the processor 140 extracts at least one reference image (the first reference image and/or the second reference image) corresponding to the ultrasound image, from the images (the first images and the second images) stored in the storage unit 120 (S304). Since the probability model may be performed by various well-known methods, a detailed description thereof will be omitted herein.

For example, the processor 140 performs contour detection processing on the ultrasound image to detect a contour (hereinafter referred to as a first contour) of the target from the ultrasound image. The contour may be detected by using an eigenvalue difference based on a mask (for example, a Sobel mask, a Prewitt mask, a Robert mask, or a Canny mask) or a structure tensor. The processor 140 performs contour detection processing on each of the images stored in the storage unit 120, to detect a contour (hereinafter referred to as a second contour) of the target from each of the images. The processor 140 detects at least one third contour corresponding to the first contour from among the second contours which are extracted from the images stored in the storage unit 120 by using the probability model. The processor 140 extracts at least one reference image (the first reference image and/or the second reference image) corresponding to the detected third contour, as the reference image of the target corresponding to the ultrasound image, from the storage unit 120.

In the above embodiment, it has been described that the reference image corresponding to the ultrasound image (i.e., the target of the ultrasound image) is extracted from the storage unit 120 by using the probability model. However, in another embodiment, the reference image corresponding to the ultrasound image (i.e., the target of the ultrasound image) may be extracted from the storage unit 120 based on the input information received from the user input unit 130.

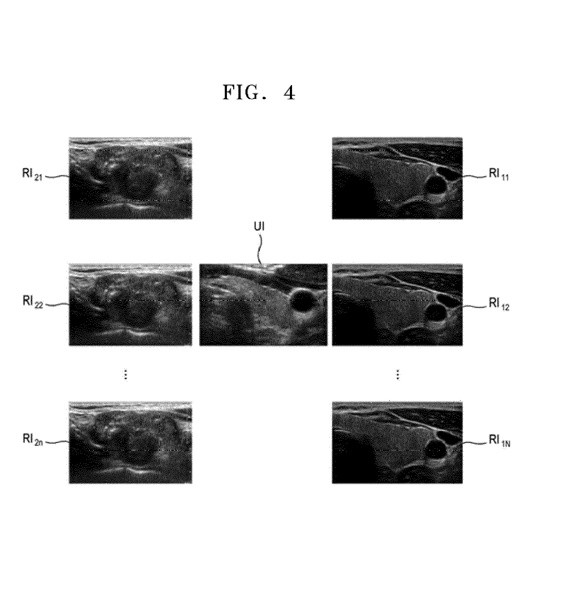

The processor 140 controls a display of the extracted reference image (S306). In an embodiment, the processor 140 operates such that the extracted first reference image or second reference image is displayed at one side with respect to the ultrasound image. As an example, as illustrated in FIG. 4, the processor 140 operates such that an extracted first reference image RI₁ᵢ (1 ≤ i ≤ N) is displayed at the right side with respect to an ultrasound image UI and an extracted second reference image RI₂ᵢ (1 ≤ i ≤ N) is displayed at the left side with respect to the ultrasound image UI. As another example, the processor 140 operates such that the extracted first reference image and second reference image is displayed at the left side or the right side with respect to the ultrasound image. In another embodiment, the processor 140 operates such that the extracted first reference image is displayed at one side with respect to the ultrasound image. In another embodiment, the processor 140 operates such that the extracted second reference image is displayed at one side with respect to the ultrasound image.

Alternatively, in response to a user command (i.e., a reference image selection command) received from the user input unit 130, the processor 140 may re-extract an image corresponding to the user command from the at least one reference image and operates such that the re-extracted image is displayed at one side with respect to the ultrasound image.

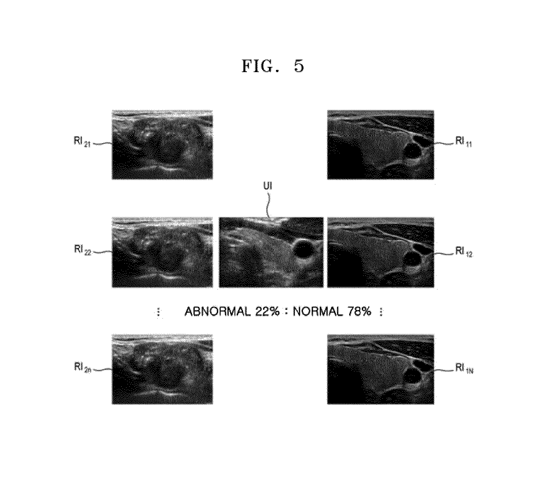

By using the ultrasound image and the extracted reference image, the processor 140 generates measurement information corresponding to the degree of normality of the target in the ultrasound image (S308). In an embodiment, the processor 140 detects the degree of similarity between the ultrasound image and the extracted reference image. For example, as illustrated in FIG. 5, the processor 140 detects the degree of similarity (normal: 78%, abnormal: 22%) between the ultrasound image and the extracted reference image. The processor 140 generates the measurement information including the detected degree of similarity. Since the degree of similarity may be detected by various well-known methods, a detailed description thereof will be omitted herein. In another embodiment, the processor 140 performs a relation analysis between the ultrasound image and the extracted reference image to generate measurement information including a relation analysis result. For example, as illustrated in FIG. 5, the processor 140 performs a relation analysis between the ultrasound image and the extracted reference image to generate measurement information including a relation analysis result (normal: 78%, abnormal: 22%). Since the relation analysis may be performed by various well-known methods, a detailed description thereof will be omitted herein.

Based on the measurement information, the processor 140 generates measurement result information corresponding to the target in the ultrasound image (S310). In an embodiment, the processor 140 compares the measurement information including the degree of similarity with a predetermined critical value, and generates measurement result information indicating that the target in the ultrasound image is normal or abnormal, depending on whether the degree of similarity is greater than the predetermined critical value. In another embodiment, the processor 140 compares the measurement information including the relation analysis result with a predetermined critical value, and generates measurement result information indicating that the target in the ultrasound image is normal or abnormal, depending on whether the relation analysis result is greater than the predetermined critical value.

The processor 140 controls a display of the measurement result information (S312). In the present embodiment, the processor 140 operates such that the measurement result information is displayed at one side of the ultrasound image.

In response to a command input through the user input unit 130, the processor 140 extracts at least one of the diagnosis information corresponding to the selected first reference image, the corresponding medical information, and the corresponding progress images, from the storage unit 120.

Referring to FIG. 1, the display unit 150 displays the ultrasound image generated by the processor 140. Also, the display unit 150 displays the reference image (the first reference image and/or the second reference image) detected by the processor 140. Also, the display unit 140 displays the measurement result information generated by the processor 130. In an embodiment, the display unit 150 displays at least one of the diagnosis information corresponding to the selected first reference image, the corresponding medical information, and the corresponding progress images.

As described above, according to the one or more of the above embodiments of the present invention, at least one of the normal reference image and the abnormal reference image corresponding to the ultrasound image of the target may be provided. Therefore, the user may use the reference image to easily determine whether the target is normal. The normal reference image and/or the abnormal reference image may be provided to the patient, thereby assisting the patient in understanding whether the target is normal.

Also, the diagnosis information, the medical information, and the progress images corresponding to the abnormal reference image may be provided, thereby facilitating the communication between the user and the patient in providing the diagnosis result and the medical treatment.

It should be understood that the exemplary embodiments described herein should be considered in a descriptive sense only and not for purposes of limitation. Descriptions of features or aspects within each embodiment should typically be considered as available for other similar features or aspects in other embodiments.

While one or more embodiments of the present invention have been described with reference to the figures, it will be understood by those of ordinary skill in the art that various changes in form and details may be made therein without departing from the spirit and scope of the present invention as defined by the following claims.

As an example, communication between the user and the patient may be facilitated by storing a plurality of normal reference images and a plurality of abnormal reference images in the storage unit 120, automatically or manually extracting the reference image, which is most similar to the ultrasound image of the target (i.e., an ultrasound image representing present state of the target) included in the subject (i.e., the patient), from the storage unit 120, and providing to the user and the patient at least one of the diagnosis information corresponding to the extracted reference image, the corresponding medical information, and the corresponding progress images.

## Claims

1. An ultrasound system comprising:
a storage unit configured to store a plurality of images corresponding to respective targets; and
a processor configured to generate an ultrasound image which depicts the target included in a subject by using ultrasound data acquired from the subject, extract at least one reference image corresponding to the ultrasound image from among the plurality of images, and control a display of the reference image extracted.

2. The ultrasound system of claim 1, wherein the processor is configured to:
detect a first contour of the target from the ultrasound image by performing contour detection processing on the ultrasound image;
detect second contours of the target from each of the plurality of images by performing contour detection processing on each of the plurality of images;
detect at least one third contour corresponding to the first contour from among the second contours by using a probability model; and
extract the reference image corresponding to the third contour detected as the reference image corresponding to the ultrasound image from the storage unit.

3. The ultrasound system of claim 1, further comprising a user input unit configured to receive from a user reference image selection information for selecting the at least one reference image from the reference image extracted, and
wherein the processor is configured to re-extract an image corresponding to the reference image selection information from the at least one reference image extracted.

4. The ultrasound system of claim 1, further comprising a user input unit configured to receive from a user reference image selection information for selecting the reference image corresponding to the ultrasound image, and
wherein the processor is configured to extract the reference image corresponding to the reference image selection information from the storage unit.

5. The ultrasound system of claim 1, wherein the processor is configured to generate measurement information corresponding to a degree of normality of the target in the ultrasound image by using the ultrasound image and the reference image extracted,
wherein the processor is configured to:
detect a degree of similarity between the ultrasound image and the reference image extracted; and
generate the measurement information including the degree of similarity detected.

6. The ultrasound system of claim 1, wherein the processor unit is configured to generate measurement information including a relation analysis result by performing a relation analysis between the ultrasound image and the reference image extracted, and
wherein the processor is further configured to generate, based on the measurement information, measurement result information corresponding to a degree of normality of the target in the ultrasound image.

7. The ultrasound system of claim 6, wherein the processor is configured to compare measurement information including a degree of similarity between the ultrasound image and the reference image extracted with a predetermined critical value, and generate the measurement result information indicating that the target in the ultrasound image is normal or abnormal depending on whether the degree of similarity is greater than the predetermined critical value.

8. The ultrasound system of claim 6, wherein the processor is configured to compare the measurement information including the relation analysis result between the ultrasound image and the reference image extracted with a predetermined critical value, and generate the measurement result information indicating that the target in the ultrasound image is normal or abnormal depending on whether the relation analysis result is greater than the predetermined critical value.

9. A method of providing a reference image, comprising:
generating an ultrasound image which depicts the target included in a subject by using ultrasound data acquired from the subject;
extracting at least one reference image corresponding to the ultrasound image from among a plurality of images stored in a storage unit configured to store the plurality of images corresponding to respective targets; and
controlling a display of the reference image extracted.

10. The method of claim 9, wherein the extracting of at least one reference image comprises:
detecting a first contour of the target from the ultrasound image by performing contour detection processing on the ultrasound image;
detecting second contours of the target from each of the plurality of images by performing contour detection processing on each of the plurality of images;
detecting at least one third contour corresponding to the first contour from among the second contours by using a probability model; and
extracting the reference image corresponding to the third contour detected as the reference image corresponding to the ultrasound image from the storage unit.

11. The method of claim 9, wherein the extracting of at least one reference image comprises receiving from a user reference image selection information for selecting the at least one reference image from the reference image extracted, and
wherein the extracting of at least one reference image comprises re-extracting an image corresponding to the reference image selection information from the at least one reference image extracted.

12. The method of claim 9, wherein the extracting of at least one reference image comprises receiving from a user reference image selection information for selecting the reference image corresponding to the ultrasound image, and
the extracting of at least one reference image comprises extracting the reference image corresponding to the reference image selection information from the storage unit.

13. The method of claim 9, further comprising generating measurement information corresponding to a degree of normality of the target in the ultrasound image by using the ultrasound image and the reference image extracted,
the generating of the measurement information comprises:
detecting a degree of similarity between the ultrasound image and the reference image extracted; and
generating the measurement information including the degree of similarity detected.

14. The method of claim 13, further comprising generating, based on the measurement information, measurement result information corresponding to a normality or abnormality of the target in the ultrasound image.

15. The method of claim 14, wherein the generating of the measurement result information comprises comparing the measurement information including the degree of similarity between the ultrasound image and the reference image extracted with a predetermined critical value, and generating the measurement result information indicating that the target in the ultrasound image is normal or abnormal depending on whether the degree of similarity is greater than the predetermined critical value.
